# EUROPÄISCHE PATENTANMELDUNG

(11) **EP 4 134 122 A1**
(43) Veröffentlichungstag der Anmeldung: **15.02.2023**
(21) Anmeldenummer: 21190538.5
(22) Anmeldetag: 10.08.2021
(51) Int. Cl.: A61M 39/28, F16K 7/06, A61B 17/3203

(54) **INSTRUMENT ZUR FLUIDAPPLIKATION UND KLEMMEINRICHTUNG**

(71) Anmelder: Erbe Elektromedizin GmbH, 72072 Tübingen (DE)
(72) Erfinder: FECH, Andreas, 72072 Tuebingen (DE); STRAUB, Frank, 72072 Tuebingen (DE)
(74) Vertreter: Rüger Abel Patentanwälte PartGmbB

(57) **Zusammenfassung**

Nach dem erfindungsgemäßen Konzept wird zur Erzeugung eines Hochdruckimpulses ein Knickschlauchventil vorgesehen, das eine im Zuleitungsschlauch (Fluidschlauch (5)) eines Instruments (2) vorgesehene Schleife (12) und eine als Mehrwegkomponente vorgesehene Schlauchknickeinrichtung (8) umfasst. Ein solches Ventil ist sehr einfach aufgebaut und gestattet die Erzeugung sehr steiler Druckanstiegsflanken bei sehr hohen Drücken bis zu 100 Bar, wie sie für die nadellose Injektion benötigt werden. Der Sterilisierungsaufwand ist minimal. Ebenso ist der Materialaufwand bei Einsatz von Einwegkomponenten minimal.

## Beschreibung

Die Erfindung betrifft ein Instrument zur Fluidapplikation sowie eine Schlauchknickeinrichtung zum Abklemmen und Freigeben eines Fluidschlauchs.

Aus der EP 3 714 926 A1 ist ein Applikationssystem bekannt, mit dessen Hilfe sich medizinische Fluide nadellos in biologisches Gewebe einführen lassen. Dazu ist in dem Instrument ein erster Fluidkanal vorgesehen, über den ein scharfer Strahl eines Fluids abgegeben werden kann, der zum Öffnen eines Injektionskanals in dem Gewebe dient. Über einen weiteren Fluidkanal wird mit geringerem Druck das medizinische Fluid, beispielsweise eine lebende Zellen enthaltende Flüssigkeit herangeführt, die dann durch den geöffneten Injektionskanal in das Gewebe eindringen kann.

Zur Ausgabe eines scharfen Fluidstrahls zur effizienten Eröffnung des Injektionskanals in dem biologischen Gewebe kommt es darauf an, dass der Anstieg des Fuiddrucks am distalen Ende des Instruments zu Beginn der Abgabe eines scharfen Injektionsstrahls möglichst steil erfolgt. Dadurch soll sichergestellt werden, dass der erzeugte Strahl von Anfang an sein volles Eindringvermögen hat und möglichst wenig Flüssigkeit in den dem Zielgewebe vorgelagerten Schichten deponiert wird und somit das Zielgewebe nicht erreicht.

**Die** EP 3 040 101 B1 offenbart eine Vorrichtung zur Erzeugung möglichst steiler Druckanstiegsflanken. Zum Sperren und Freigeben von unter Druck stehenden Fluidkanälen sind Ventile vorgesehen. Diese müssen entweder im Ganzen sterilisierbar ausgebildet sein oder sie müssen als Einwegkomponenten ausgebildet sein.

Unabhängig davon müssen sie jedenfalls geeignet sein, einen erheblichen Fluiddruck von bis zu 100 Bar schlagartig freigeben oder auch sperren zu können.

Zum Öffnen und Freigeben von Fluidkanälen sind sogenannte Quetschventile bekannt. Ein Beispiel offenbart die US 9 095 656 B2**.** Das dort beschriebene Quetschventil wird durch einen flexiblen Leitungsabschnitt gebildet, der innerhalb eines Druckbehälters angeordnet ist. Dieser Druckbehälter dient auch als Energiequelle für die Erzeugung eines Fluidstrahls. Durch die Anordnung eines flexiblen Schlauchs in dem Druckbehälter herrscht im Leitungsinneren stets annähernd der gleiche Druck wie außerhalb der Zuleitung. Somit muss der Ventilantrieb lediglich die Rückstellkraft der Wandung des flexiblen Leitungselements überwinden. Es ergeben sich somit lediglich geringe Schließkräfte. Die Freigabe des Fluidkanals erfolgt durch die Rückstellkraft der Wandung und wird von dem Fluiddruck durch die geringe Druckdifferenz zwischen dem Druck innerhalb des Fluidkanals und dem Druck außerhalb des Schlauchs nur wenig unterstützt. Dies kann die Steilheit des Druckanstiegs beim Öffnen des Ventils vermindern.

Es sind auch sogenannte Knickventile bekannt. Dazu offenbart die US 2 540 364 ein mit mehreren Fingerschlaufen versehenen Schlauch. Die Fingerschlaufen können auf Finger und Daumen einer Hand eines Bedieners aufgezogen werden, der dann durch einfache Handbewegung den Schlauch knicken oder freigeben kann.

Weitere mechanisierte Vorrichtungen zum Knicken und Freigeben eines Schlauchs sind aus der GB 1 012 565**,** der DE 698 15 825 T2**,** der EP 2 133 610 A1 oder der EP 2 130 562 A2 bekannt.

Die DE 60 2004 009 494 T2 offenbart eine Vorrichtung, die mittels zweier Exzenterscheiben an einem Schlauch mehrere hintereinander angeordnete Knickstellen definiert, die Ventile bilden. Diese Knickstellen bzw. Ventile werden durch Kurvenscheiben alternierend geöffnet und geschlossen, um aus einem Hochdruckfluidreservoir kleine Fluidportionen auszulassen und mit niedrigem Druck auszugeben. Ein einfaches magnetisch betätigtes Knickventil offenbart außerdem die US 6 056 260**.**

Es ist Aufgabe der Erfindung, ein Konzept für ein Instrument und ein zugehöriges Ventil anzugeben, mit dem sich ein unter hohem Druck stehendes Fluid impulsweise mit steiler Druckanstiegsflanke abgeben lässt und das einen lediglich geringen Aufwand erfordert.

Diese Aufgabe wird einerseits mit dem Instrument nach Anspruch 1 und andererseits mit der Schlauchknickeinrichtung nach Anspruch 9 gelöst:

Erfindungsgemäß weist das Instrument einen ersten Fluidschlauch auf, der an eine erste Fluidquelle anschließbar ist, um das Instrument mit unter hohem Druck stehenden Fluid zu versorgen. Der erste Fluidschlauch des Instruments wird selbst als Ventil genutzt. Er wird dazu in eine Schlauchknickeinrichtung nach Anspruch 9 eingeführt, die eine Betätigungseinrichtung aufweist, um eine von dem Fluidschlauch gebildete Schleife gezielt abzuknicken oder freizugeben. Zur Erleichterung des Einführens einer Schleife des Fluidschlauchs in die Schlauchknickeinrichtung, kann an dem Fluidschlauch gemäß Anspruch 1 ein Halter angeordnet sein, um den Fluidschlauch in einer Schleife zu halten. Die Schleife kann dann wie ein Stecker gehandhabt und in einen entsprechenden Kanal der Schlauchknickeinrichtung eingesetzt werden. Dieses Konzept trennt die leicht sterilisierbaren oder auch mit geringem Aufwand stets neu bereitstellbaren und somit als Einwegteile bereitstellbaren instrumentenseitigen Komponenten von nicht oder nur schwer sterilisierbaren und nur mit hohem Aufwand jeweils neu bereitstellbaren geräteseitigen Komponenten. Jedes Knickventil wird somit insgesamt von einer geräteseitigen Komponente, nämlich der Schlauchknickeinrichtung, sowie durch den ersten Fluidschlauch des Instruments gebildet.

Das dem Instrument über den ersten Fluidschlauch zugeleitete Fluid kann unter hohem Druck stehen und impulsweise mit jeweils steiler Druckanstiegsflanke geliefert werden, um in biologischem Gewebe einen Injektionskanal zu bilden. Das Instrument kann einen zweiten Fluidschlauch aufweisen, dessen proximales Ende an eine zweite Fluidquelle anschließbar ist. Diese kann unter einem anderen, insbesondere einem niedrigeren Druck stehen, um nach Öffnen des Injektionskanals medizinisches Fluid abzugeben, das von dem Instrument bzw. dem Fluidapplikator in den geöffneten Injektionskanal und somit in das biologische Gewebe eingetragen wird.

Während der erste Fluidschlauch von dem Halter in einer Schleife gehalten ist, um in die Schlauchknickeinrichtung eingeführt zu werden, ist der zweite Fluidschlauch vorzugsweise knick- und schleifenfrei ausgebildet bzw. geführt. Es ist jedoch auch möglich, auch den zweiten Fluidschlauch durch eine Schlauchknickeinrichtung zu führen, um seine Knickstelle als Ventil zu nutzen.

Die von dem Halter festgelegte Schleife weist vorzugsweise zwei Schenkel auf, die von dem Halter etwa parallel zueinander gehalten sind. Die Länge L der Schleife, insbesondere die Länge ihrer Schenkel, ist dabei vorzugsweise größer als der Abstand der beiden Schenkel untereinander, der ihre Breite B festlegt. Die Länge L und die Breite B sind dabei so aufeinander abgestimmt, dass die Schleife im Ruhezustand offen ist. Sie knickt und schließt somit mit lediglich einem Knick, wenn die Breite B durch die Schlauchknickeinrichtung verringert wird.

Der Halter ist auf dem ersten Fluidschlauch vorzugsweise fest, das heißt unverschiebbar angeordnet. Damit bestimmt der Halter die Größe der Schleife und somit auch die Position der Schleife gegenüber der Betätigungseinrichtung der Schlauchknickeinrichtung, sobald der Halter mit der Schleife wie ein Stecker in den Kanal der Schlauchknickeinrichtung eingesteckt ist. Somit ist eine ordnungsgemäße Funktion sichergestellt.

Anstelle des Halters können jedoch auch andere Mittel zur Begrenzung der Einstecktiefe der Schleife in den Kanal vorgesehen sein. Beispielsweise kann in Einsteckrichtung hinter der Betätigungseinrichtung ein Anschlagmittel, beispielsweise eine Wand, vorgesehen sein, an die die Schleife anstößt, wenn sie in den Kanal eingeschoben ist. In diesem Fall bildet diese Wand ein Positioniermittel zur Festlegung der Schleifenposition relativ zu der Betätigungseinrichtung. In solche Schlauchknickeinrichtungen können auch von Hand zu einer Schleife geformte Abschnitte eines Fluidschlauchs eingelegt werden, der keinen Halter aufweist. In Verbindung mit der Schlauchknickeinrichtung nach Anspruch 8 können auch Instrumente genutzt werden, die nicht nach Anspruch 1 ausgebildet sind.

Bei Schlauchknickeinrichtungen, denen ein solches vorgenanntes Positioniermittel fehlt, kann der an dem ersten Fluidschlauch des Instruments vorgesehene Halter die Funktion dieses Positioniermittels übernehmen. Der Halter findet seine Anlage dann an einer in Einsteckrichtung vor der Betätigungseinrichtung liegenden Wand, die als Steckeranschlag zugleich die Einstecktiefe der Schleife begrenzt.

Weitere Details vorteilhafter Ausführungsformen der Erfindung sind Gegenstand der Figuren der Zeichnung, der zugehörigen Beschreibung sowie von Unteransprüchen. Es zeigen:
Figur 1 eine Behandlungseinrichtung mit einem Instrument und einer Schlauchknickeinrichtung gemäß der Erfindung,
Figur 2 die Schlauchknickeinrichtung und einen Teil des Fluidschlauchs des Instruments nach Figur 1 in ausschnittsweiser schematisierter Darstellung,
Figur 3 den Fluidschlauch und die Schlauchnickeinrichtung nach Figur 2 in perspektivischer getrennter Darstellung,
Figur 4 die Anordnung nach Figur 2 in perspektivischer Darstellung,
Figur 5 eine etwas abgewandelte Ausführungsform einer Schlauchknickeinrichtung mit Fluidschlauch in offenem Zustand,
Figur 6 die Schlauchknickeinrichtung nach Figur 5 und den Fluidschlauch des Instruments in geschlossenem Zustand,
Figur 7 eine abgewandelte Ausführungsform der Schlauchknickeinrichtung bei geschlossenem Fluidschlauch,
Figur 8 die Schlauchknickeinrichtung nach Figur 7 bei offenem Fluidschlauch,
Figur 9 eine abgewandelte Ausführungsform eines Instruments sowie eines versorgenden Geräts mit Schlauchknickeinrichtung in perspektivischer Darstellung,
Figur 10 ein Instrument mit Schlauchknickeinrichtung in schematisierter Darstellung und
Figur 11 eine abgewandelte Ausführungsform der Schlauchknickeinrichtung.

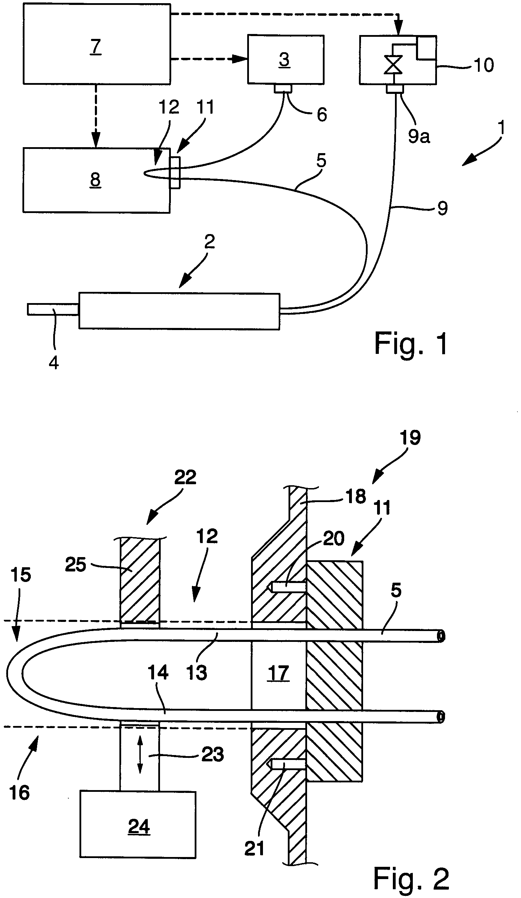

In Figur 1 ist eine Einrichtung 1 zur Behandlung eines Patienten mittels Injektion medizinischer Flüssigkeit veranschaulicht. Zu der Einrichtung 1 gehören ein zum Beispiel von Hand zu führendes Instrument 2 und weitere Geräte und Baueinheiten zur Versorgung des Instruments 2 mit einer oder mehreren Flüssigkeiten. Zu diesen Baueinheiten gehört mindestens eine erste Fluidquelle 3, die beispielsweise unter hohem Druck stehendes Fluid aufweist, zum Beispiel Natriumchloridlösung. Der Druck ist ausreichend hoch, um einen dem Instrument 2 vorgesehenen Fluidapplikator mit unter Injektionsdruck stehendem Fluid zu versorgen. Der Injektionsdruck ist so hoch, dass ein aus dem Fluidapplikator 4 austretender Fluidstrahl in biologisches Gewebe eindringt und dort einen Injektionskanal öffnet. Außer physiologischer Kochsalzlösung kann die Fluidquelle 3 auch andere biologisch verträgliche Fluide enthalten und bereitstellen.

Das Instrument 2 ist über einen ersten Fluidschlauch 5 mit der Fluidquelle 3 verbunden oder verbindbar. Dazu kann der Fluidschlauch 5 an seinem proximalen Ende mit einem Stecker 6 versehen sein. Sein distales Ende ist hingegen vorzugsweise fest, bedarfsweise aber auch lösbar mit dem Instrument 2 verbunden.

Die Fluidquelle 3 kann darauf eingerichtet sein, unter Druck stehendes Fluid über den Stecker 6 an das Instrument 2 permanent abzugeben. Die Fluidquelle 3 kann jedoch auch Ventile oder sonstige Steuerungsorgane enthalten, um das unter Druck stehende Fluid gezielt frei zu geben oder zu sperren. Zur Steuerung der Fluidquelle 3 kann eine Steuereinrichtung 7 vorgesehen sein.

Der Fluidapplikator 4 benötigt das von der Fluidquelle 3 gelieferte, unter hohem Druck stehende Fluid, vorzugsweise impulsweise mit steilen Druckanstiegsflanken. Zur Bereitstellung und Erzeugung solcher Druckimpulse ist eine Schlauchknickeinrichtung 8 vorgesehen, die von der Steuereinrichtung 7 gesteuert werden kann. Die Schlauchknickeinrichtung 8 bildet zusammen mit einem Abschnitt des Fluidschlauchs 5 ein Hochdruckventil zum Sperren und Freigeben des Fluids in dem Fluidschlauch 5.

Das Instrument 2 kann über einen zweiten Fluidschlauch 9 mit einer zweiten Fluidquelle 10 verbunden sein, die ein zweites Fluid bereitstellt, insbesondere ein als Medikament dienendes Fluid, eine Zellaufschwemmung oder dergleichen. Die zweite Fluidquelle kann das Fluid mit einem niedrigeren Druck dauernd oder impulsweise bereitstellen. Zur Steuerung der Fluidabgabe kann die zweite Fluidquelle 10 ein Ventil enthalten und von der Steuereinrichtung 7 gesteuert sein. Der Fluidschlauch 9 kann an die Fluidquelle 10 über einen Stecker 9a angeschlossen sein.

Die Schlauchknickeinrichtung 8 und der Fluidschlauch 5 bilden gemeinsam ein Hochdruckventil, das in Figur 2 in Prinzipdarstellung veranschaulicht ist. Der Fluidschlauch 5 besteht aus einem geeigneten üblichen Kunststoff, wie beispielsweise PU, PFA, FEP, KPFA, KEEP, PVC, Silikon, PA, TPE-S oder irgendeinem anderen geeigneten Kunststoff. Es können faserverstärkte oder armierte Kunststoffschläuche Anwendung finden.

Der Schlauch 5 ist in einem Halter 11 gefasst, der, wie Figur 1 erkennen lässt, zwischen dem proximalen und dem distalen Ende des Fluidschlauchs 5 vorzugsweise in der Nähe des Steckers 6 angeordnet ist. Der Halter 11 ist auf dem Fluidschlauch 5 fest angebracht, um einen Abschnitt des Schlauchs 5 in einer Schleife 12 zu halten. Diese Schleife weist zwei zueinander im Wesentlichen parallele Schenkel 13, 14 auf, die an einem Schleifenkopf 15 ineinander übergehen. Der Fluidschlauch weist eine solche Steifigkeit auf, dass er, wenn die beiden Schenkel 13, 14 aufeinander zu bewegt werden, an der Stelle mit der größten Krümmung, nämlich an dem Schleifenkopf 15, unter Absperrung seines Lumens knickt, ohne dass das Lumen an anderer Stelle durch Quetschung kollabiert.

Der Halter 11 bildet zusammen mit der Schleife 12 einen Stecker. Die Schleife 12 des so gebildeten Steckers lässt sich in einen in Figur 2 gestrichelt abgegrenzt dargestellten Einsteckereich 16 einführen. Den Eingang des Einsteckbereichs 16 bildet eine schlitzartige Öffnung 17, die in einer vorderen Wand 18 der Schlauchknickeinrichtung 8 ausgebildet ist. Die Wand 18 ist somit zugleich ein Positioniermittel 19 für den Stecker bzw. den Halter 11. An dem Halter 11 können Stifte 20, 21 vorgesehen sein, die in entsprechende Öffnungen der Wand 18 passen, um den Halter 11 an der Wand 18 zu fixieren und zu positionieren. Anstelle der Stifte 20, 21 können auch andere Verbindungs- und Positioniermittel vorgesehen sein, beispielsweise Permanentmagnete oder andere mechanische oder nicht mechanische Rastmittel.

Der Einsteckbereich 16 ist der Raum, den die Schleife 12 in entspanntem Zustand einnimmt. Er erstreckt sich durch eine Betätigungseinrichtung 22 hindurch, die dazu geeignet ist, die Schenkel 13, 14 entgegen ihrer eigenen Federspannung aufeinander hin zu bewegen. Dabei ist die Betätigungseinrichtung vorzugsweise näher an dem Schleifenkopf 15 angeordnet, als an dem Halter 11. Die Betätigungseinrichtung 22 kann ein bewegliches Element 23 aufweisen, das geeignet ist, den Schenkel 14 in Richtung des Schenkels 13 zu drängen. Entsprechend kann das Element 23 mit einer Antriebseinrichtung 24 in Verbindung stehen. Das Element 23 kann linear beweglich oder auch schwenkbeweglich angeordnet sein.

Dem Element 23 gegenüberliegend kann ein weiteres Element 25 angeordnet sein, das als festes Widerlager ausgebildet sein kann. Alternativ kann das Element 25 auch dem Element 23 entgegen beweglich angeordnet und mit einer entsprechenden Antriebseinrichtung verbunden sein (nicht dargestellt) .

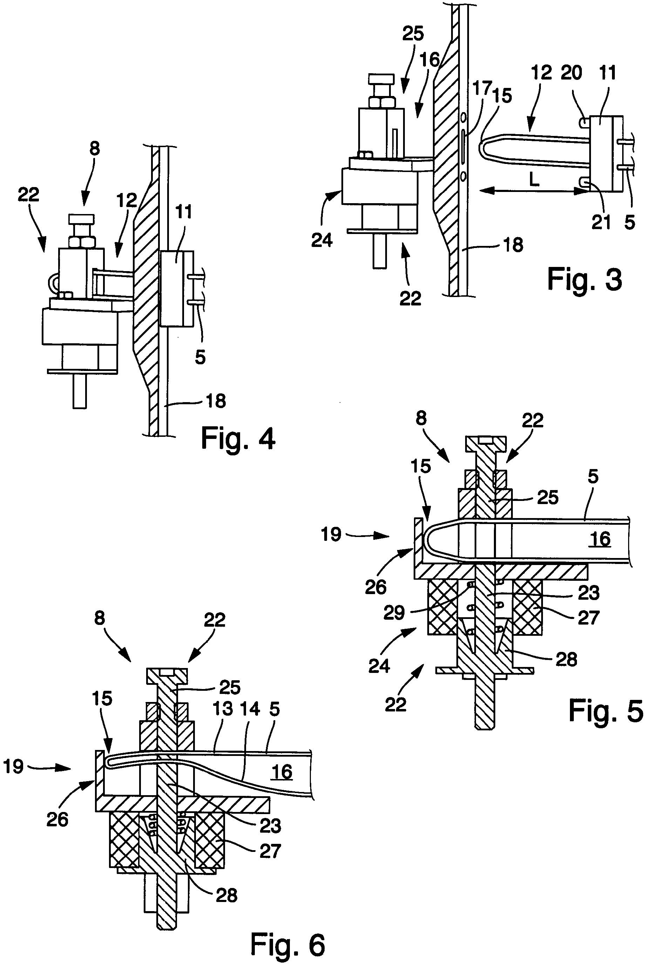

Figur 3 veranschaulicht den in Figur 2 dargestellten Sachverhalt nochmals perspektivisch. Ersichtlicherweise wird der Einsteckbereich 16 wiederum von der Öffnung 17 und einer entsprechenden Öffnung zwischen dem als Widerlager dienenden Element 25 und dem in Figur 3 durch die Antriebseinrichtung 24 verdeckten beweglichen Element 23 gebildet.

Figur 4 veranschaulicht die in die Schlauchknickeinrichtung 8 eingesteckte Schleife 12. Der Halter 11 liegt an der Wand 18 an. Die Schleife 12 erstreckt sich durch die Betätigungseinrichtung 22. Der Schleifenkopf 15 liegt von der Wand 18 aus gesehen hinter der Betätigungseinrichtung 22.

Bei der insoweit beschriebenen Ausführungsform bestimmt der Halter 11 die Einstecktiefe der Schleife 12 in die Schlauchknickeinrichtung 8. Die Wand 18 ist somit (zusammen mit dem Halter 11) das Positioniermittel für die Schleife 12.

Es ist jedoch auch möglich, die Einstecktiefe der Schleife 12 durch einen Anschlag 26 für den Schleifenkopf 15 festzulegen, wie es aus Figur 5 und 6 hervorgeht. Der Anschlag 26 bildet dann wiederum das Positioniermittel 19. Bei einer solchen Ausführungsform der Schlauchknickeinrichtung 8 kann auf den Halter 11 verzichtet oder der Halter 11 kann in größerem Abstand zu der Frontwand 18 angeordnet werden. Auch können Halter 11 Anwendung finden, die auf dem Fluidschlauch 5 nicht ortsfest sondern repositionierbar (z.B. verschiebbar) angeordnet sind.

Die Antriebseinrichtung 24 kann als Magnetantrieb ausgebildet sein. Beispielsweise kann dazu eine Spule 27 vorgesehen sein, in deren Mitte ein Anker 28 angeordnet ist, der mit dem beweglichen Element 23 fest verbunden ist. Eine Feder 29 kann dann dazu dienen, den Anker 28 und mit ihm das Element 23 in Ruheposition außerhalb des Einsteckbereichs 16 zu halten.

Die Betätigungseinrichtung 22 und die Schleife 12 bilden zusammen ein Ventil. Der fluidseitige Teil des Ventils wird durch die Schleife 12 gebildet, die zu dem Instrument 2 gehört. Der vom Fluid nicht berührte Teil des Ventils wird durch die Schlauchknickeinrichtung 8 gebildet, die als separates Gerät ausgebildet sein oder zu einem Gerät gehören kann, das die Steuereinrichtung 7 sowie die Fluidquelle 3 und 10 umfasst.

Zum Injizieren von Fluid in biologisches Gewebe arbeitet die insoweit beschriebene Einrichtung 1 wie folgt:

Nach dem Anschluss des Instruments 2 an die Fluidquellen 3 und 10 wird der aus dem Halter 11 und der Schleife 12 gebildete Stecker in die Schlauchknickeinrichtung 8 eingefügt, wie es die Figur 2, 4 oder 5 veranschaulicht. Somit ist die Einrichtung 1 grundsätzlich arbeitsbereit.

Nach dem Einschalten der Steuereinrichtung 7 erfasst die Steuereinrichtung 7 den korrekten Anschluss des Instruments 2 an die Fluidquellen 2, 10 sowie das Einsetzen der Schleife 12 in die Schlauchknickeinrichtung 8. Die Steuereinrichtung 7 schließt nun das aus der Schleife 12 und der Schlauchknickeinrichtung 8 gebildete Ventil, wie Figur 6 veranschaulicht, indem die Spule 27 bestromt wird. Dadurch wird der Anker 28 in die Spule 27 hineingezogen und dadurch das Element 23 gegen das als Widerlager dienende Element 25 gedrängt. Die beiden Schenkel 13, 14 werden aufeinander zu bewegt, so dass der Fluidschlauch 5 an dem Schlauchkopf 15 knickt. Der Fluidkanal ist in diesem Zustand abgesperrt und es kann kein Hochdruckfluid zu dem Instrument 2 vordringen.

Das Widerlager 24 ist vorzugsweise so angeordnet, dass der Schenkel 13 im Wesentlichen unverformt bleibt. Der Bewegungsweg des Elements 23 ist vorzugsweise so festgelegt, dass es in zurückgezogenem Zustand den Querschnitt des Schenkels 14 im Wesentlichen undeformiert belässt und, wenn es maximal in Richtung auf das Widerlager 24 zu bewegt ist, von diesem einen Abstand hält. Der Abstand ist vorzugsweise wenigstens doppelt so groß wie der Durchmesser des Fluidschlauchs 5. So wird ein Quetschen des Fluidschlauchs 5 vermieden. Mit anderen Worten, der Schlauchquerschnitt bleibt im Bereich der Betätigungseinrichtung im Wesentlichen kreisförmig, sein Lumen ist hier offen (nicht zusammengequetscht).

Vor dem Schließen dieses Ventils kann der Fluidschlauch 5 mit Fluid gespült werden, um im Fluidschlauch 5 und in dem Instrument 2 sowie dem Applikator 4 vorhandene Luft zu beseitigen. Dazu gibt die Steuereinrichtung vor dem Schließen des von der Schleife 12 gebildeten Ventils Fluid aus der Fluidquelle 3 vorzugsweise mit vermindertem Druck kurzzeitig frei.

Soll nun einem Patienten ein medizinisches Behandlungsfluid appliziert werden, wird das unter hohem Druck stehende Fluid 3 von dem Instrument 2 und dem Fluidapplikator 4 zunächst zum Öffnen eines Injektionskanals genutzt. Dazu schaltet die Steuereinrichtung 7 kurzzeitig die Spule 27 stromlos. Der Anker 28 wird dadurch nicht mehr angezogen. Die Feder 29 drängt den Anker 28 aus der Spule 27, womit die Position nach Figur 5 erreicht wird. Der Knick an dem Schleifenkopf 15 wird beseitigt und schlagartig gelangt Fluid mit hohem Druck zu dem Fluidapplikator 4. Der hohe Fluiddruck unterstützt den Schlauch bei seiner Öffnungsbewegung. Der Fluidapplikator 4 gibt einen scharfen Fluidstrahl ab, der in biologischem Gewebe einen Injektionskanal öffnen kann.

Obwohl sich mit diesem Konzept an dem Fluidapplikator 4 bereits ein ausgesprochen steiler Druckanstieg erzielen lässt, kann der Druckanstieg noch steiler gestaltet werden. Ist der Anker 28 beispielsweise als permanentmagnetischer Anker ausgebildet, kann das Herausführen des beweglichen Elements 23 aus dem Einsteckbereich 16 noch beschleunigt werden, in dem die Spule 27 zum Öffnen des Ventils nicht stromlos gemacht, sondern mit umgekehrter Polarität bestromt wird. Auch kann eine zwischen den Schenkeln 13, 14 wirksame Feder das Auseinanderschwenken der Schenkel 13, 14 beschleunigen.

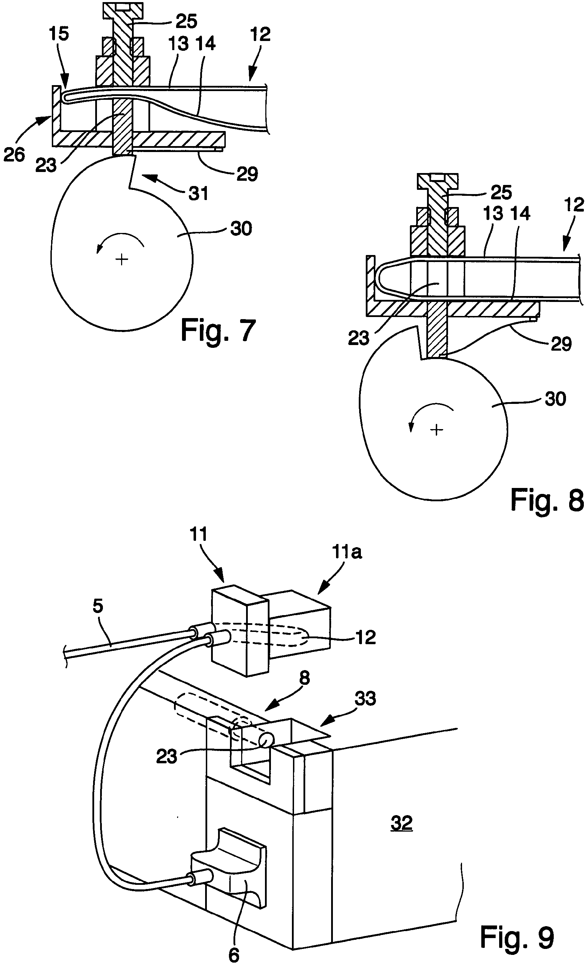

Eine weitere effiziente Möglichkeit dazu veranschaulicht Figur 7 und Figur 8. Das bewegliche Element 23 wird hier durch eine Nockenscheibe 30 betätigt, die eine oder mehrere Nocken aufweisen kann. Beispielsweise kann der Umfang der Nockenscheibe 30 einer Spirale folgen und am Ende derselben einen Rücksprung 31 aufweisen. Die Nockenscheibe 30 kann mit ihrem spiralförmigen Umfang mit dem beweglichen Element 23 in Anlage stehen, das als Kurvenfolger arbeitet. Ein geeignetes Federmittel, beispielsweise die als Blattfeder ausgebildete Feder 29, hält das bewegliche Element 23 mit der Nockenscheibe 30 in Anlage.

Zum Schließen des Ventils ist beispielsweise ein geeigneter Motor, zum Beispiel ein Schrittmotor vorgesehen, der die Nockenscheibe 30 dreht, bis das Element 23 den höchsten Punkt des Umfangs der Nockenscheibe 30 erreicht hat. Soll das Ventil nun geöffnet werden, dreht der Schrittmotor die Nockenscheibe 30 wie Figur 8 veranschaulicht etwas weiter, so dass das bewegliche Element 23 den Rücksprung 31 erreicht und überschreitet. Unter der Wirkung der Rückfederkraft der Schenkel 13 und 14 der Schleife 12 und unter der Wirkung der Feder 29 springt das Element 23 praktisch unverzögert in seine Ruhestellung, so dass die Öffnung des Ventils mit größtmöglicher Geschwindigkeit erfolgt.

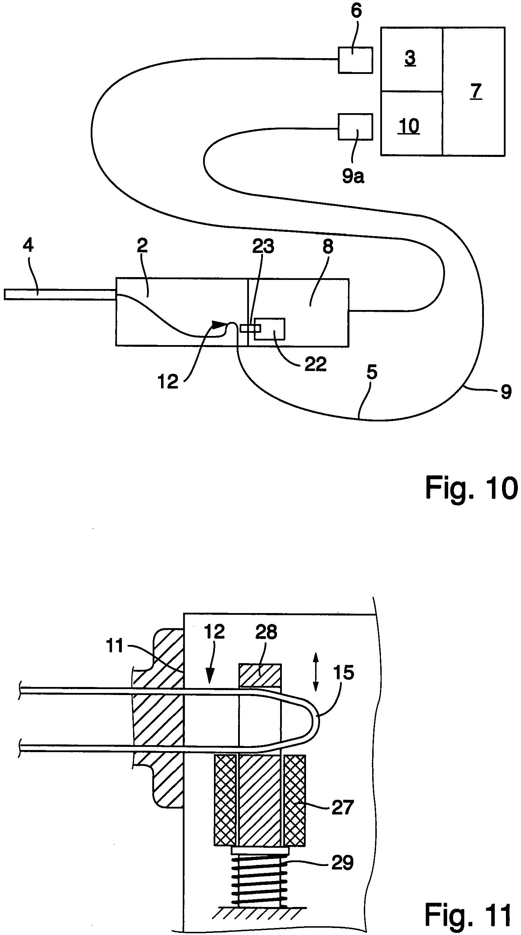

Eine weitere Variante der Betätigungseinrichtung 22 veranschaulicht Figur 11. Dort weist der Anker 28 einen Schlitz auf, dessen Breite geringfügig größer ist als der Durchmesser des Fluidschlauchs 5 und somit einerseits die seitliche Führung der Schleife 12, andererseits aber auch die Bewegung des Schlauchs in Öffnungs- und Schließrichtung erlaubt. Damit definiert der Anker 28 einen Teil des Einsteckbereichs 16.

Die Feder 29 drängt den Anker 18 in die dargestellte Freigabeposition. Bei Bestromung der Spule 27 wird der Anker 28 in Figur 11 nach unten gezogen, so dass die Schleife 12 an dem Schlauchkopf 15 knickt. Ansonsten gilt die Beschreibung der vorigen Ausführungsbeispiele entsprechend.

Figur 9 veranschaulicht ein Gerät 32, das sowohl die Fluidquellen 3 und 10, sowie auch die Schlauchknickeinrichtung in sich vereint. Der Halter 11 fasst die Schleife 12 in einem Teilgehäuse 11a, das auch eine Anlagefläche für einen der beiden Schenkel 13 oder 14 aufweist. Der Anlagefläche gegenüberliegend weist das Teilgehäuse 11a eine Öffnung auf, durch die ein Stößel in das Teilgehäuse 11a eindringen kann, um die Schleife 12 zu knicken. Der Stößel kann wiederum durch ein bewegliches Element 23 gebildet sein, das von einem geeigneten Aktor, beispielsweise einem Elektromagneten oder einer Nockenscheibe, betätigt wird. In dem Teilgehäuse 11a kann ein Federmittel angeordnet sein, das zwischen den Schenkeln 13, 14 angeordnet ist und auf den Schenkel 14 einwirkt. Das Federmittel kann eine Kraft aufbringen, die dem Knicken des Schlauchkopfs entgegen wirkt.

Zur Aufnahme des Teilgehäuses 11a in der Schlauchknickeinrichtung weist dieses ein Aufnahmefach 33 auf, das funktional dem Einsteckbereich 16 entspricht. Das Aufnahmefach kann, wie Figur 9 veranschaulicht, nach oben offen oder auch als Einsteckkanal ausgebildet sein.

Eine weitere wichtige Abwandlung des erfindungsgemäßen Konzepts veranschaulicht Figur 10. Dort ist die Schlauchknickeinrichtung 8 als Mehrwegkomponente eines Einweginstruments 2 ausgebildet. Die Schlauchknickeinrichtung 8 enthält die Betätigungseinrichtung 22 und ist lösbar mit dem Handgriff des Instruments 2 verbunden. Das bewegliche Element 23 der Betätigungseinrichtung 22 kann in verbundenem Zustand in die Einwegkomponente hineinragen und dort auf die Schleife 12 einwirken, um diese zu knicken oder freizugeben. Die Steuerung der Betätigungseinrichtung 22 erfolgt über eine entsprechende Steuerverbindung zu der Steuereinrichtung 7, z.B. über eine nicht weiter veranschaulichte Leitung.

Nach dem erfindungsgemäßen Konzept wird zur Erzeugung eines Hochdruckimpulses ein Knickschlauchventil vorgesehen, das eine im Zuleitungsschlauch (Fluidschlauch 5) eines Instruments 2 vorgesehene Schleife 12 und eine als Mehrwegkomponente vorgesehene Schlauchknickeinrichtung 8 umfasst. Ein solches Ventil ist sehr einfach aufgebaut und gestattet die Erzeugung sehr steiler Druckanstiegsflanken bei sehr hohen Drücken bis zu 100 Bar, wie sie für die nadellose Injektion benötigt werden. Der Sterilisierungsaufwand ist minimal. Ebenso ist der Materialaufwand bei Einsatz von Einwegkomponenten minimal.

### Bezugszeichen:

- 1: Einrichtung
- 2: Instrument
- 3: erste Fluidquelle
- 4: Fluidapplikator
- 5: erster Fluidschlauch
- 6: Stecker
- 7: Steuereinrichtung
- 8: Schlauchknickeinrichtung
- 9: zweiter Fluidschlauch
- 10: zweite Fluidquelle
- 11: Halter
- 11a: Teilgehäuse
- 12: Schleife
- 13, 14: Schenkel der Schleife 12
- 15: Schleifenkopf
- 16: Einsteckbereich
- 17: Öffnung
- 18: Frontwand
- 19: Positioniermittel
- 20, 21: Stifte
- 22: Betätigungseinrichtung
- 23: bewegliches Element
- 24: Antriebseinrichtung
- 25: Element
- 26: Anschlag
- 27: Spule
- 28: Anker
- 29: Feder
- 30: Nockenscheibe
- 31: Rücksprung
- 32: Gerät
- 33: Aufnahmefach

## Patentansprüche

1. Instrument (2) mit
einem Fluidapplikator (4),
einem ersten Fluidschlauch (5), der sich von einem proximalen, an eine erste Fluidquelle (3) anschließbaren Ende bis zu dem Fluidapplikator (2) erstreckt,
mit einem Halter (11), der an dem ersten Fluidschlauch (5) angeordnet ist, um diesen in einer Schleife (12) zu halten.

2. Instrument nach Anspruch 1, **dadurch gekennzeichnet, dass** der Fluidapplikator (4) einen zweiten Fluidschlauch (9) aufweist, der an seinem proximalen Ende (9a) an eine zweite Fluidquelle (10) anschließbar ist.

3. Instrument nach Anspruch 2, **dadurch gekennzeichnet, dass** sich der zweite Fluidschlauch (9) von seinem proximalen Ende (9a) knick- und schleifenfrei bis zu dem Fluidapplikator (4) erstreckt.

4. Instrument nach einem der vorstehenden Ansprüche, **dadurch gekennzeichnet, dass** die Schleife (12) eine Länge (L) aufweist, die größer ist als ihre Breite (B) .

5. Instrument nach einem der vorstehenden Ansprüche, **dadurch gekennzeichnet, dass** die Schleife (12) zwei in dem Halter (11) zueinander parallel gehaltene Schenkel (13, 14) aufweist.

6. Instrument nach einem der vorstehenden Ansprüche, **dadurch gekennzeichnet, dass** die Schleife (12) in dem Halter (11) unverschiebbar gehalten ist.

7. Instrument nach einem der vorstehenden Ansprüche, **dadurch gekennzeichnet, dass** der Halter (11) wenigstens ein Verbindungsmittel (20, 21) zur Verbindung mit einer Schlauchknickeinrichtung (8) aufweist.

8. Instrument nach einem der vorstehenden Ansprüche, **dadurch gekennzeichnet, dass** das Instrument (2) als Einmalinstrument ausgebildet ist.

9. Schlauchknickeinrichtung (8)
mit einem Einsteckbereich (16), in den eine Schleife (12) eines Fluidschlauchs (5) in einer Einsteckrichtung einführbar ist,
mit einer Betätigungseinrichtung (22), die an dem Einsteckbereich (16) angeordnet ist, um die Schleife (12) gesteuert zu verformen oder freizugeben.

10. Schlauchknickeinrichtung nach Anspruch 9, **dadurch gekennzeichnet, dass** der Einsteckbereich (16) einen Schlitzquerschnitt aufweist.

11. Schlauchknickeinrichtung nach einem der Ansprüche 9 oder 10, **dadurch gekennzeichnet, dass** sie ein Positioniermittel (19) zur Festlegung der Position der Schleife (12) relativ zu der Betätigungseinrichtung (22) aufweist.

12. Schlauchknickeinrichtung nach einem der Ansprüche 9 bis 11, **dadurch gekennzeichnet, dass** das Positioniermittel (19) ein in Einsteckrichtung hinter der Betätigungseinrichtung (22) angeordneter Anschlag (26) ist.

13. Schlauchknickeinrichtung nach einem der Ansprüche 9 bis 11, **dadurch gekennzeichnet, dass** das Positioniermittel (19) ein in Einsteckrichtung () vor der Betätigungseinrichtung (22) angeordneter Steckeranschlag (18) ist.

14. Schlauchknickeinrichtung nach einem der Ansprüche 9 bis 13, **dadurch gekennzeichnet, dass** die Schlauchknickeinrichtung (8) als sterilisierbare Einrichtung ausgebildet ist.

15. Einrichtung umfassend ein Instrument (2) nach einem der Ansprüche 1 bis 8 und Schlauchknickeinrichtung (8) nach einem der Ansprüche 9 bis 14.
